# EUROPEAN PATENT SPECIFICATION

(11) **EP 3 530 260 B1**
(45) Date of publication and mention of the grant of the patent: **01.11.2023**
(21) Application number: 17862142.1
(22) Date of filing: 27.09.2017
(51) Int. Cl.: A61K 8/04, A61K 8/67, A61K 8/64, A61K 8/97, A61K 8/41, A61K 8/02, A45D 44/00, A45D 44/22, A61Q 19/00

(54) **METHOD FOR PREVENTING OXIDATION OF ANTIOXIDANT MATERIAL USING APTAMER, MATERIAL, AND USE THEREOF**
VERFAHREN ZUR VERHINDERUNG DER OXIDATION VON ANTIOXIDATIVEM MATERIAL UNTER VERWENDUNG VON APTAMER, MATERIAL UND VERWENDUNG DAVON
PROCÉDÉ DE PRÉVENTION DE L'OXYDATION D'UN MATÉRIAU ANTIOXYDANT À L'AIDE D'UN APTAMÈRE, MATÉRIAU ET UTILISATION DE CELUI-CI

(30) Priority: 19.10.2016 WO PCT/KR2016/011740
(43) Date of publication of application: 28.08.2019
(73) Proprietor: Nexmos Co., Ltd., Yongin-si, Gyeonggi-do 16827 (KR)
(72) Inventor: SON, In Sik, Seongnam-si Gyeonggi-do 13614 (KR)
(74) Representative: Berggren Oy
(86) International application number: PCT/KR2017/010756
(87) International publication number: WO 2018/074763

(56) References cited:
- EP-A1- 1 082 956
- WO-A1-2009/031788
- WO-A1-2016/029139
- WO-A1-2017/150891
- US-A1- 2007 112 064
- US-A1- 2013 196 915
- JYOTI BALA ET AL: "In vitro selected RNA aptamer recognizing glutathione induces ROS mediated apoptosis in the human breast cancer cell line MCF 7", RNA BIOLOGY, vol. 8, no. 1, 1 January 2011 (2011-01-01) , pages 101-111, XP055688465, ISSN: 1547-6286, DOI: 10.4161/rna.8.1.14116
- TYLER D. AMES ET AL: "A Eubacterial Riboswitch Class That Senses the Coenzyme Tetrahydrofolate", CHEMISTRY AND BIOLOGY., vol. 17, no. 7, 1 July 2010 (2010-07-01), pages 681-685, XP055688453, GB ISSN: 1074-5521, DOI: 10.1016/j.chembiol.2010.05.020
- MAUREEN MCKEAGUE ET AL: "Development of a DNA aptamer for direct and selective homocysteine detection in human serum", RSC ADVANCES, vol. 3, no. 46, 1 January 2013 (2013-01-01), pages 24415-24422, XP055688481, DOI: 10.1039/c3ra43893g
- L. BASELGA-ESCUDERO ET AL: "Resveratrol and EGCG bind directly and distinctively to miR-33a and miR-122 and modulate divergently their levels in hepatic cells", NUCLEIC ACIDS RESEARCH, vol. 42, no. 2, 27 October 2013 (2013-10-27), pages 882-892, XP055688483, ISSN: 0305-1048, DOI: 10.1093/nar/gkt1011
- BARRITA, JOSE LUIS SILENCIO ET AL.: 'Antioxidant Role of Ascorbic Acid and His Protective Effects on Chronic Diseases' OXIDATIVE STRESS AND CHRONIC DEGENERATIVE DISEASES - A ROLE FOR ANTIOXIDANTS, [Online] 2013, pages 449 - 484, XP055414186 ISBN: 978-953-51-1123-8 Retrieved from the Internet: <URL:https://www.intechopen.com/books/oxida tive-stress-and-chronic-degenerative-diseas es-a-role-for-antioxidants/antioxidant-role -of-ascorbic-acid-and-his-protective-effect s-on-chronic-diseases>

## Description

### [Technical Field]

The present disclosure relates to a method for preventing the oxidation of an antioxidant using a nucleotide aptamer, its material, and its use.

### [Background Art]

Ascorbic acid (Vitamin C) being one of representative components among antioxidants is widely used in medicine, cosmetics, food and beverage industries due to the antioxidant activity via neutralization of its free radical. Advantages of AA when it is used as food additive and/or medical active component were reported as that inhibiting the acute oxidation process of pulmonary cell resulted from the inhalation of ZnO NPs which can be generated in industrial disaster (tire and rubber plant, cosmetics-manufacturing plant) by supplying drinking water added AA. It was known that in these industries which cost tens of trillion won, Vitamin C is commonly used as a major solution.

AA can be easily degraded essentially by oxidation due to its antioxidant ability. Major factors affecting the oxidation of AA are temperature, pH, oxygen, metal ion, light, enzyme, etc. In the industries utilizing AA as a major component, since such oxidation and degradation properties affect both storage period and effect, this has long been recognized as the matter in hand. Therefore, many studies and costs are invested on development and finding new and better method as well as the understanding on the oxidative degradation of AA in these industries.

Meanwhile, there are many reasons for promoting aging, but reactive oxygen species (ROS) is considered to be one of the major causes. This ROS is indispensably produced in energy metabolism, immune response, etc., and is generated by inevitable stimulus caused by external harmful environment. ROS is highly reactive to cause DNA degeneration, induction of excessive signal transmission, protein denaturation, etc. in the body, resulting in a series of reactions accumulating adverse health effects. However, in these harmful conditions, homeostasis have been elaborately maintained by external antioxidants (uric acid, vitamin C, vitamin E, etc.) or endogenous antioxidant enzymes (glutathione peroxidase, superoxide dismutase, catalase, etc.) which presents in the body. However, the aging of the antioxidant system due to the endogenous aging and the accumulation of ROS by the continuous and noxious stimuli may break this balance, promoting aging, causing various diseases such as skin diseases, skin cancer, arteriosclerosis, and thrombosis (Laure Rittie et al., Aging Research Reviews, 1, 705-720, 2002; Cutler RG, Annals of the New York Academy of Sciences, 1055, 93-135, 2005).

Accordingly, there is a growing interest in antioxidants that inhibit the formation of reactive oxygen species(ROS) or removing ROS. Antioxidants can be divided into those that are naturally present in the body (endogenous antioxidant) and those that are administered externally (exogenous antioxidant). Antioxidants that are naturally present in the body include enzymes such as superoxide dismutase (SOD), glutathione, peroxidase, and catalase. Externally administered antioxidants include phytochemicals such as kaempferol, catechin, and genistein; vitamin E, vitamin C, and beta carotene; and minerals such as selenium.

Cells are attacked by free radicals, oxygen free radicals, and etc. caused by ultraviolet A (UVA) and ultraviolet B (UVB) irradiated from sunlight, pollutants, stress, smoking, drinking, and fatty foods. If the proper protection from these materials is not achieved, the cells will age or die. In the case of skin, the production of materials such as collagen and elastin is reduced or denatured by these materials, causing the skin to lose its elasticity and resulting in wrinkles. In order to prevent this, it is known that it is important to prevent aging of skin by applying a preparation containing antioxidants such as vitamins A, C, and E to the skin and absorbing it into the skin by preventing oxidation from the harmful environmetal factors. However, vitamin C( L-ascorbic acid) is easily oxidized in the air and by sunlight, and thus its antioxidant effect disappears. Therefore, there is a problem in manufacturing various formulations having a long storage period.

In addition, vitamin C having very high reducing power reacts to materials having high oxidation potential very sensitively and vitamin C is oxidized rapidly. It is well-known fact that when vitamin C is oxidized the efficiency thereof is deteriorated since water has high oxidation potential, vitamin C is sensitively reacted, and thus rapidly oxidized.

Therefore, there has long been a need for new method and/or materials inhibiting oxidation of antioxidants comprising vitamin C. US 2007/112064 A1 discloses that nucleic acid, DNA or RNA, can be used to protect sensitive molecules, including antioxidants (e.g. vitamin C, E, lipid, oil oestrogen etc.) from oxidative degradation. Successful prevention of degradation of vitamin C is shown in the examples and thus stabilised formulations can be food, cosmetics and so on.

Also, preventing the oxidation of antioxidants using aptamer is a safe and innovative new approach compared to existing methods, and this can be applied to various industries so as to prepare so that the effect is maximized. In particular, it will be a catalyst for transforming existing chemical-based cosmetics, nutritional supplements, and food markets into a market based on DNA (BIO). Also, it can be newly used in various industries. In the future, it is expected to provide explosive increase in DNA market and innovative solution.

### [Prior Patent Document]

Korean Patent No.: 10-1197677

### [Disclosure]

### [Technical Problem]

The present disclosure has been made in view of the above needs, and an object of the present disclosure is to provide a method for preventing oxidation of an antioxidant using aptamer.

Another object of the present disclosure is to provide material preventing oxidation of antioxidant.

Still another object of the present disclosure is to provide a method for functionalized hydrogel which contains aptamer preventing oxidation of an antioxidant and has a function of controlling the release rate and composition of active cosmeceutical ingredient according to the amount of a specific substance released from the skin.

Another object of the present disclosure is to provide uses as a cosmetic, a hair nutrient, a hairy dye, a topical skin treatment agent and a nutritional supplement by using a material having antioxidant-inhibiting action.

Another object of the present disclosure is to provide a material which maintains antioxidant function of Vitamin C for a longer time by maintaining the reduced state thereof.

Another object of the present disclosure is to provide a method for preparing foods and beverages through a method for maintaining the reduced state of vitamin C in a liquid such as water for a longer time.

### [Technical Solution]

In order to achieve the objects as described above, the present disclosure provides a method for treating an aptamer to antioxidant to reduce oxidizing rater of the antioxidant and maintain the reduction power thereof.

In the present disclosure, the antioxidant is vitamin C.

The aptamer is a single-strand DNA nucleotide whose sequence is shown in SEQ ID NO: 1 or SEQ ID NO. 2. However, in addition to such aptamer, aptamers which achieve the desired effect of the present disclosure and have other sequences as demonstrated by the examples, of the present disclosure could also be found.

In another embodiment of the present disclosure, the aptamer inhibits the oxidation of the second and third OH groups of the lactone ring of vitamin C.

In the present disclosure, an aptamin is defined as a complex of antioxidant and aptamer. For example, an aptamin C means a complex of vitamnin C and aptamer.

### 1. Application example of cosmetics using aptamer-based hydrogel

The present disclosure provides an aptamer for reducing oxidation rate of the antioxidant The aptamer of the present disclosure is preventing oxidation of vitamin C, and the aptamer includes a base sequence as set forth in SEQ ID NO: 1 or SEQ ID NO: 2.

Further, the present disclosure also provides a method for producing an aptamer-trapped hydrogel, including the steps of: a) binding an amine group to an aptamer of the present disclosure; b) silanizing hydroxyl group of the hydrogel monomer with 3-glycidoxypropyltrimethoxysilane (3-GPTMS) and then binding the aptamer-bound amine group to the epoxy group; and c) polymerizing the hydrogel monomer.

In one embodiment of the present disclosure, a method includes the steps of binding biotin to the aptamer-bound amine group of the step a), reacting them with a particle having streptavidin, and then mixing the particles having the aptamer with the hydrogel monomer during the hydrogel polymerization.

In another embodiment of the present disclosure, a method preferably, but not limited to, chemically binds an amine group or a carboxyl group attached to the aptamer to the hydroxyl group of the hydrogel.

Further, the present disclosure provides an aptamer trapped hydrogel produced by the method of the present disclosure.

In one embodiment of the present disclosure, the hydrogel is such that the aptamer is attached to a surface of hydrogel and a specific ingredient is attached to an end of the aptamer. The specific ingredient is preferably, but not limited to, a component having skin aging resistance, wrinkle removal, whitening, and moisturizing effect.

The specific ingredient of the present disclosure may be any raw material used in cosmetics, regardless of the kind of extracts or active ingredients. Examples thereof may include various extracts such as, for whitening, a green tea extract, a licorice extract, a mulberry extract, a mulberry root extract, a golden extract, a pueraria extract, a red ginseng extract, for preventing aging, an apricot extract, an oil extract, an orange extract, a lemon extract, a bamboo extract, a guava extract, a rosemary extract, a cornus officinalis extract, a lingshi mushroom extract, a ginkgo extract, a gleditschia australis thorn extract, a paeonia lactiflora root extract, for moisturizing, a quince extract, a white lotus flower extract, a paprika extract, an aloe extract, a cylindrica extract, a seaweed extract, for anti-oxidation, a carrot extract, a soybean extract, a grapefruit seed extract, a grape seed extract, a portulaca oleracea extract, for improving wrinkles, caviar, pomegranate, a ginseng extract, for skin reproduction, a peach extract, a cnidium officinale MAKING extract, for treating atopy, a centella asiatica extract, a chamomile extract, an adriatic root extract, a sophora flavescens extract, an angelica extract, for treating acne, a peppermint extract, a saururus chinensis extract, a heartleaf houttuynia extract, a peony extract, for anti-inflammatory or anti-bacteria, pyrolignous liquor, a dandelion extract, a calendula extract, a phellodendron amurense extract, a trifoliate orange extract, a golden extract, a fennel extract, a compuri extract, for shrinking pores, a castanea crenata shell extract, a green tea extract, for moisture, glycerin, panthenol, hyaluronic acid, ceramide, beta-glucan, for whitening, albutin, vitamin C, whitense, retinol, astaxanthin, resveratinol, polyphenol, for elasticity, elastin, collagen, coenzyme Q10, effectin, EGF, for anti-infective anti-bacterial agent, propolis, allantoin, phytostan, infra acid, antioxidant vitamin E (natural tocopherol) ROE (a rosemary oil extract), a grapefruit seed extract.

Further, the present disclosure provides a cosmetic composition including the aptamer-trapped hydrogel of the present disclosure.

Further, the present disclosure provides a method for controlling the release of the skin active material according to the amount of the target material released from the skin, including the steps of applying an aptamer-trapped hydrogel of the present disclosure to the skin to cause the aptamer binding to an ingredient from the hydrogel, so as to penetrate into the skin, binding the ingredient- aptamer comples to the target substances from the skin, and releasing a active ingredient from hydrogel.

In one embodiment of the present disclosure, the target substance form the skin is preferably, but not limited to, ATP whose levels are changed according to the skin condtions.

Because aptamers can effectively stablize the antioxidant vitamin C, by preventing its spontaneous oxidation, it is expected that its cosmeceutical effects for skin whitening and wrinkle improvemnts could be improved when they are slowly released from the hydrogels.

The use of cosmetic raw material/materials using the aptamer-hydrogel of the present disclosure is summarized as follows.

As widely known, materials used as the main ingredients of functional cosmetics such as vitamin C, peptide, and retinol are very unstable . When the materials are exposed to air and light they are easily oxidized and lose their antioxidant function quickly ,. These materials are captured by the aptamer to inhibit the materials to bind to the oxygen (oxidation), thereby giving the role of sustaining the materials as stable as possible (continuous function).

The release rate of the active cosmeceutical ingredients is controlled by aptamers. Since most hydrogels have non-compact structure, the permeability is high for many kinds of materials so that the materials contained therein are easily released. It has been experimentally proven that when aptamer which reacts with specific active ingredient is included in the hydrogel, the release rate of the materials can be controlled by controlling the binding force of the aptamer to the materials.

Aptamer can regulate the release rate of the active ingredients according to the amount of the specific substance released from the skin. This technique, called Aptasensing, is a method that can be used for various purposes such as cosmetics and therapy by releasing the necessary materials to the skin according to the condition of the skin after detecting the various states of the skin. There is an implementable system that detects ATP released at a different concentration depending on the temperature of the skin, or detects, e.g., cytokines, by the aptamer, then the active ingredients are released according to the skin condition.. Thus, it allows an appropriate amount of various active ingredients to be released by sensing the skin condtions so as to be designed to control the duration time or reduce unnecessary overload to the skin.

A three-dimensional structure of aptamers such as single strand DNA or RNA confers great flexibility and specificity toward its target molecule. Even though it is similar to an antigen-antibody reaction, its size is much smaller, and its activity can be controlled by various methods. Thus, it has the advantages of easy production and storage as compared to antibodies. Further, the aptamer may be synthesized to bind to a chemical substance (vitamin) having a very small size unlike an antibody. Since it is produced by chemical synthesis, it is easy to maintain its effect constantly.

Vitamin C is a water-soluble six-carbon compound, which includes a reduced form in which C-3 and C-4 (3- and 4-) are formed in dihydroxy and semidehydroascorbic acid and dehydroascorbic acid in which those carbons are oxidized respectively.

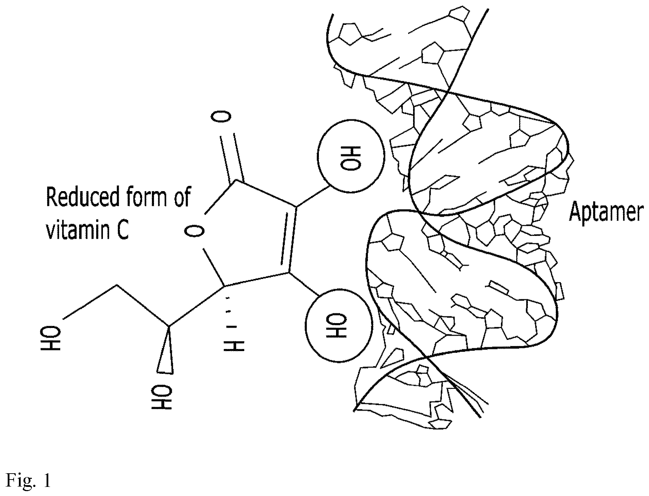

The reduced state of vitamin C is maintained through the hydrogen bonding of the hydroxyl group of vitamin C and the base constituting the aptamer (RNA or DNA) of the present disclosure (*See* FIG. 1).

Vitamin C, which is in a reduced state in combination with the aptamer of the present disclosure, can be used for nutritional supplements or cosmetic compositions of various formulations of hydrogel-type or cream-type, including, e.g., collagen, elastin, hyaluronic acid, and peptides.

The present disclosure also includes a method for slowly releasing vitamin C according to various conditions of the skin through aptamer (aptasensing) which differently reacts depending on the state of the skin or external stimuli (for example, ultraviolet rays, skin temperature, and acidity). Such examples include a method that the bound vitamin C is released when the structure of the aptamer is changed depending on the irradiation ultraviolet on the skin, and that vitamin C is released when the amount of ATP is changed depending on the change of the skin temperature or acidity.

Also, the present disclosure includes uses in various formulations (SERUM, Gel, lotion, cream, toner, Mask pact, etc.) cosmetics, hair nutrients and colorants comprising the complex of the aptamers and antioxidant of the present disclosure as an effective ingredient.

In one embodiment of the present disclosure, said cosmetic composition preferably includes at least one of collagen, elastin, hyaluronic acid and peptide, but is not limited thereto.

### 2. Nutritional Additive using aptamer or aptamer-antioxidation component complex

The present disclosure provides an nutritional additive composition comprising a complex of aptamer and antioxidant or an aptamer alone as an effective component. In the present disclosure, the aptamer includes a base sequence as set forth in SEQ ID NO: 1 or SEQ ID NO: 2. This aptamer is preventing oxidation of vitamin C, that is the nutritional additive according to the present disclosure.

Examples of vitamin suitable for nutritional additive of the present disclosure include vitamin A, vitamin C, vitamin D, vitamin E, vitamin E, vitamin K, vitamin B6, vitamin B12, thyamine, riboflavin, biotin, folic acid, niacin, pantothenic acid, mixtures thereof, etc. Examples of suitable mineral nutrients to be included in the nutritional additive composition include those comprising at least one elements selected from sodium, potassium, calcium, magnesium, phosphorous, sulphur, chlorine, iron, copper, iodine, zinc, selenium, manganese, chromium, molybdenium, fluorine, cobalt, and mixtures thereof.

Various herbs can also be used as the nutritional additive. In general, the herb is selected from those having medicine or diet additive property. Generally, the herb is a part of aromatic plant or plant.

In addition, the present disclosure provides a method for delaying oxidation rate by binding aptamer to antoxidant and maintaining the reduction state of the antioxidant.

Hereinafter, the present disclosure is illustrated.

The present disclosure can use antioxidants being very unstable to oxidation, as the nutritional additive by binding them with the aptamer. The aptamer includes a base sequence as set forth in SEQ ID NO: 1 or SEQ ID NO: 2. This aptamer is preventing oxidation of vitamin C, that is the nutritional additive according to the present disclosure.

Through this, it is ensured that the effects required by the materials is able to be maintained as much as possible. Also, it is possible to maximize the effect by allowing the release under the target condition through aptasensing.

In addition, the present disclosure provides a complex of the antioxidant component and aptamer which is developed in accordance with the antioxidant component thereof, or separately provides the active aptamer and the antioxidant components.

In the present disclosure, said antioxidant is vitamin C.

### 3. Food and beverage and food composition using a complex of aptamer and/or antioxidant

The present disclosure provides a beverage composition comprising a complex of an aptamer and antioxidant as an effective component, or comprising an aptamer alone. The aptamer includes a base sequence as set forth in SEQ ID NO: 1 or SEQ ID NO: 2. This aptamer is preventing oxidation of vitamin C, that is the nutritional additive according to the present disclosure.

In one embodiment of the present disclosure, said composition comprises preferably further comprises at least one of collagen, elastin, hyaluronic acid and peptide, but is not limited thereto.

Also, the present disclosure provides a food composition comprising the aptamer of the present disclosure and/or antioxidant as an effective ingredient.

In one embodiment of the present disclosure, said food composition comprises preferably further comprises at least one of collagen, elastin, hyaluronic acid and peptide, but is not limited thereto.

In one embodiment of the present disclosure, said food composition is preferably confectionaries, candies, dairy products, gums, soy sauces, breads, or ice cream, but is not limited thereto.

Also, the present disclosure provides a method for preparing foods by adding the aptamer of the present disclosure to the foods.

In another embodiment of the present disclosure, the food or beverage composition makes the bound vitamin C to release when the food or beverage is absorbed into the body and the structure of aptamer is changed, or allows the vitamin C to release by changing the structure of the aptamer bound thereto when the amount of ATP is changed depending on the change of environment, but is not limited thereto.

### [Effects of Invention]

As can be seen from the present disclosure, it is expected that the aptamer of the present disclosure has a preventive effect of spontaneous oxidation of antioxidant vitamin C, and the hydrogel trapped by the aptamer of the present disclosure have functions controlling the release rate through the aptamer, and controlling the release component depending on the amount of specific material released from a skin

The aptamer of the present disclosure has antioxidation effect of the antioxidant vitamin C, the aptamer alone or the complex of the aptamer and antioxidant can be used in the various formulations of functional cosmetics and dietary supplements for oral use, etc., by maintaining the reduction state of vitamin C using the aptamer selectively binding to vitamin C (ascorbic acid), for example, and maintaining the antioxidant function thereof for a longer time. The continued and maximized antioxidation effect can be expected even with a small amount of vitamin C by using the aptamer selectively binding to vitamin C.

Further, as can be seen from the present disclosure, the present disclosure can be used in various health beverage, antioxidation beverage, antioxidation food, etc. by maintaining the reduction state of vitamin C using the aptamer selectively binding to vitamin C (ascorbic acid), etc., and maintaining the antioxidant function thereof for a longer time.

Also, preventing the oxidation of antoxidant by using the aptamer is new safe and innovative approach of concept compared to existing methods, and this can be applied to various industries and thus the preparation is possible so as to obtain the effect. In particular, this will be an initiator which dramatically changes the existing cosmetics, nutritional additive and food markets based on chemicals into the DNA (BIO)-based markets. In the future, it is expected that an explosive increase and innovative solution in DNA market will be provided.

### [Description of Drawings]

FIG. 1 shows a drawing maintaining the reduction state of vitamin C through the hydrogen bonding of the hydroxyl group of vitamin C with bases constituted in an aptamer (RNA or DNA),
FIGS. 2-4 shows an experimental data related to aptamers A, B and C, wherein FIG. 2 is a drawing showing the DHA detection-inhibiting analysis, which is a time-lapse graph of DHA detection (OPDA fluorescence) under the presence of each of aptamers. It shows that the graphs for aptamers B and C are somewhat different from the control, and aptamer A is not departed from the control and thus No. 22 does not interfere with the detection analysis.
FIG. 3 is a drawing showing the oxidative protection of AA by the aptamers for the oxidant, and overlays of controls and aptamer A AT 10.3 µM CuSO₄, 103.3 µMNaIO₄, 10.3 µM ,H₂O₂, and 10.3 µM TEMPOL, and FIG. 4 is a drawing showing the aptamer titration for AA wherein titration data shows that the oxidative protection of AA is increased as the aptamer concentration is increased.
FIG. 5 is a drawing representing the AA protection over the time at vitaminwater^{®}, which is a graph comparing the degradation of AA in the presence or absence of aptamer A.
FIGS. 6-14; Test data related to aptamers # 1, 2 and 3, wherein FIGS. 6-8 are the drawings that three aptamers of the present disclosure prevent the oxidation of vitamin C by hydrogen peroxide water, and FIGS. 9-14 are the graphs of dissociation constant (KD) of AA (ascorbic acid) and DHA (dehydroascorbic acid) of aptamer of the present disclosure.

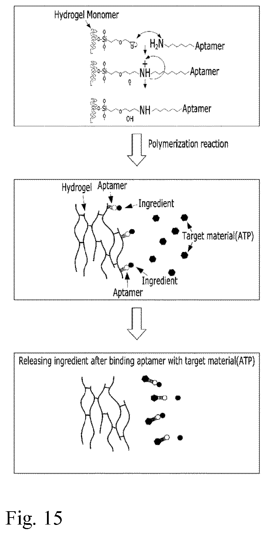

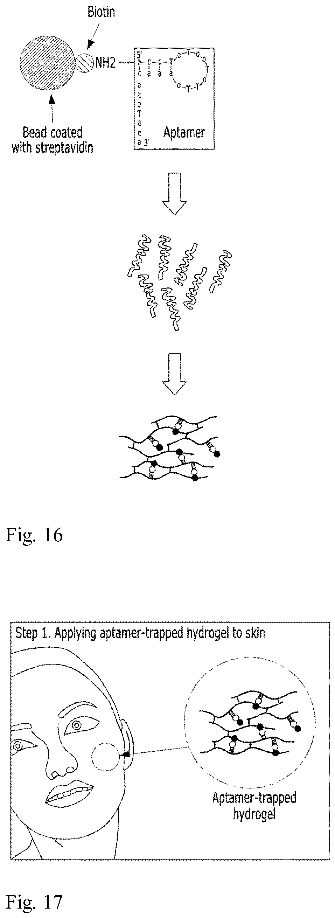

FIGS. 15 and 16 are drawings showing the preparation method of functional smart hydrogel using the aptamer of the present disclosure.

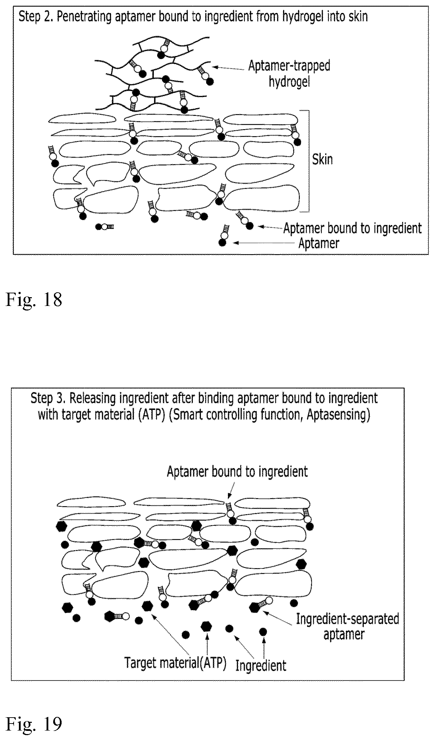

FIGS. 17-19 are drawings showing the procedures for aptasensing the hydrogel trapped by the aptamer of the present disclosure.

### [Best Mode]

Hereinafter, the present disclosure will be described in detail with reference to non-limiting Examples. Provided that, the following Examples are intended to illustrate the present disclosure and the scope of the present disclosure is not to be construed as being limited by the following Examples.

In the present disclosure, in order to make that a target (AA) is not to be rapidly oxidized, all buffers and solutions are stirred for one hour in Chelex^{®} 100 resin (BioRad), filtered through 0.2 µm filter (Sarstedt), and N2 gas (Praxair) was spared for 10 minutes to prepare it by using a molecular biological grade of water (Phenix Research) wherein accidental metals are removed.

### Example 1: DNA aptamer selection and sequencing

### Ascorbic Acid SELEX:

9 rounds of SELEXs for ascorbic acid (AA) were preformed using DNA library (BasePair library) composed of ~ 10¹⁵ unique oligonucleotides. The buffer composition used is as follows: 50 mM Sodium Acetate pH 5.5 (Sigma), 1 mM MgCl2 (Sigma), 0.05% Tween 20 (Sigma), 1% BSA (Sigma) and 1 mM glutathione (Sigma). The stringency of the SELEX was changed by deceasing the binding time of aptamer to the target, changing the buffer composition and decreasing the concentration of the target at the free molecular elution. The negative selection for DNA was performed so as to remove aptamers binding to ascorbic acid in an oxidation state from an enriched library.

### Example 2: Fluorescence analysis for oxidation products of ascorbic acid

The oxidation of ascorbic acid was measured reversely by modifying the method as described in Vislisel et al. (Vislisel, J.M., Schafer, F.Q. and Buettner, G.R. (2007) Analytical biochemistry, 365, 31-39) and detecting the oxidized product, dehydroascorbic acid (DHA).

In summary, aptamers (41.3 µM) were incubated with AA (10.3 µM) at 4X concentration for 30 minutes at room temperature before adding an oxidant (10.3 µM for CuSO4 (EM Science) and H2O2 (Sigma), 103.3 µM for TEMPOL (Sigma) and NaIO4 (Sigma). Before adding OPDA dye (Sigma) it was incubated for 10 minutes at room temperature after adding oxidizing agent sample. It was read under the condition of excitation at 345 nm; emission at 425 nm with SpectraMax^{®} i3X plate reader (Molecular Devices) for 45 minutes at 60 second intervals until the control is converged immediately after adding dye (954.6 µM) sample. To confirm that screening data represent AA protection, and there was no interference of the oxidation product (DHA) or assay dye (OPDA), a fluorescence analysis was repeated with DHA (10.3 µM) using the selected aptamer cultivated at the site of AA. All analyses were performed by modifying 50 mM sodium acetate (Sigma), 1% BSA (Sigma), 0.05% Tween 20 (Sigma), 1 mM, MgCl2 (Sigma) adjusted to pH 5.5. All fluorescence analyses were performed at Black 384-well plate (greiner bio-one). Each sample was repeated 3 times.

### Example 3: Aptamer titration for AA

In order to determine the effective concentration of the optimal aptamer (A), it was titrated to the AA (10.3 µM). Relative concentrations of aptamers for AA were 10x, 5x, 2x, 1x, 0.5x, 0.25x and 0.1x (1.03 µM aptamer at 103 µM). All aptamers / AA mixtures were incubated at room temperature for 30 min before the addition of 10.3 µM CuSO4, and the sample was incubated at room temperature for additional 10 min before the addition of 954.6 µM OPDA. The plate was read for 10 min at ex: 345 nm; em: 425 nm, and collected data every 60 seconds. Each sample was repeated three times.

### Example 4: Measurement of aptamer affinity

Materials such as ascorbic acid and dehydroascorbic acid were purchased from Sigma-Aldrich (St. Louis, MO). Affinity measurements for aptamer to AA and DHA were performed using MST (microscale-thermophoresis) (Jerabekwillemsen M et al. Assay and drug development technologies. 2011; 9 (4): 342-53). In summary, Cy5-conjugated aptamer (5 nM) was made 16 consecutive dilutions and incubation of target (AA or DHA) up to 1.53 nM at 50 uM. Before loading with a scanning capillary tube (4 uL / tube), the mixture was incubated for 15 minutes. The fluorescence of each sample was measured using Monolith NT.115 MST during exposure to a thermal gradient using Monolith NT. 115 MST instrument (NanoTemper Technologies), and a binding curve was made. Each analysis was repeated three times.

### Example 5: Mass Spectrometry

### Sample preparation:

Aptamer A (50 uM) was incubated to vitaminwater^{®} (kiwi-strawberry) (Glaceau, Coca-cola). The samples were incubated at room temperature and 10 uL was collected at 0 h, 1, 2, 4, 8, 24, 48, 72, 96 and 168h. Ascorbic acid (Cerilliant Cat # V-038) and dehydroascorbic acid (Sigma-Aldrich Cat # 261556) controls were injected before and after each time in order to monitor AA and DHA stability during LC-MS/MS assay. Each sample and control were spiked with an ascorbic acid internal standard C13 (Toronto Research Chemicals Cat#A786992) at a final concentration of 200 ng/mL. AA stock solution (Cerilliant 1 mg/mL in acetonitrile: H2O, 50:50) was diluted with 10 ug/mL and AAC13 to 1000 ng/mL. 10 uL of AA (2 ug/mL) was diluted with 90 uL of AA-C13 to 1000 ng / mL to obtain final concentrations of 1000 ng/ml and 900 ng/ml from AA and AA-C13, respectively. DHA control group was prepared with a final concentration of 1 ug/ml with 900 ng/ml of AA-C13. 10 uL of sample at each time was diluted 1 : 200 in the same internal standard solution and analyzed by LC-MS/MS.

### LC-MS / MS parameters (MRM):

Acquisition was performed with an ABSciex QTRAP 6500 (ABSciex, Foster City, CA, USA) coupled to an Eksigent µUHPLC (Eksigent, Redwood City, Calif., USA) and equipped with an electrospray interface with a 50 µm iD capillary. Analyst 1.6.3 software was used to adjust the device and to process and recognize data in a "low mass hardware configuration". Optimized MRM parameters were used to monitor ascorbic acid, ascorbic acid-C13 and DHA. The samples were injected by loop over-filling by 2 µL loop and analyzed by LC-MS/MS. The separation was performed on Gemini C18 from Phenomenex 50 mm x 0.5 mm which is maintained at 45°C. During the 3.5 min LC gradient, the mobile phases consisted of solvent A (10 mM TBA and 15 mM acetic acid in water) and solvent B (10 mM TBA and 15 mM acetic acid in methanol) at a flow rate of 30 µL/min. The gradient was started at 100 : 0 A : B. TBA was used as an ion-pairing reagent, and each sample was repeated three times.

The results of the above Examples are as follows.

### DNA aptamer selection

Biological information analysis of the abundant library produced by the ELEX method obtained a candidate aptamer, and the ability to protect AA from oxidation from the top 20 aptamers was screened.

### Oxidative protection of AA by candidate aptamers

### Screening of aptamer pools

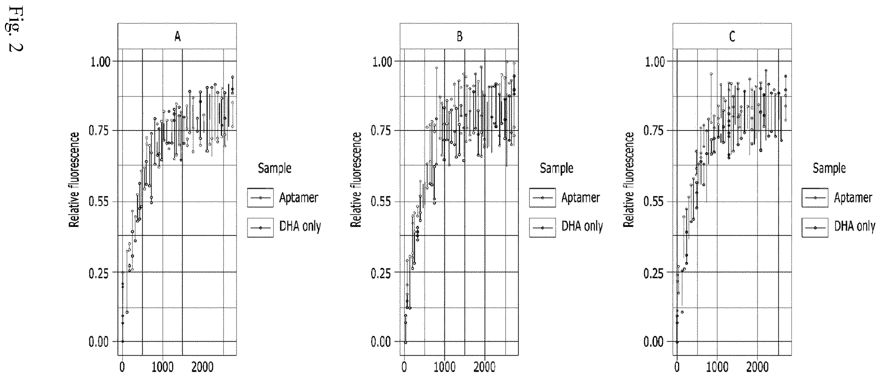

The accumulation/detection of DHA in this assay indicates the oxidation of AA. Thus, if an aptamer protects AA from oxidation, the resulting signal will be lower than the positive (no aptamer) control. An overlay graph of each candidate appamer mixed with AA and oxidizer, positive control (AA plus oxidizer) and negative control (AA minus oxidizer) was shown in FIG. 2, and each tile represents a plurality of aptamer candidates. Among twenty (20) aptamers, they have a distinct difference less than positive control (aptamers A, B, and C), and thus three (3) aptamers having clear protection effect of AA (aptamers A, B, and C), and these reduced the oxidation by 35± 13%, 43 ± 9% and 25 ± 8%, respectively, for Nos. 22, 27 and 44 (n = 3).

### Determination of aptamer that interferes with detection analysis

To determine whether the top 3 atamers (A, B, and C) obtained from screening oxidized with the detection assay by the aptamers themselves, DHA being AA product was analyzed. Aptamer A did not deviate from the DHA control graph, and this suggests that the aptamer does not interfere with the detection method. However, the aptamers B and C showed a slight difference from the control graph, suggesting that the data generated in screening of these aptamers are unreliable (FIG. 2). To create reliable data, all additional experiments were performed using only aptamer A.

### Aptamer screening for oxidants

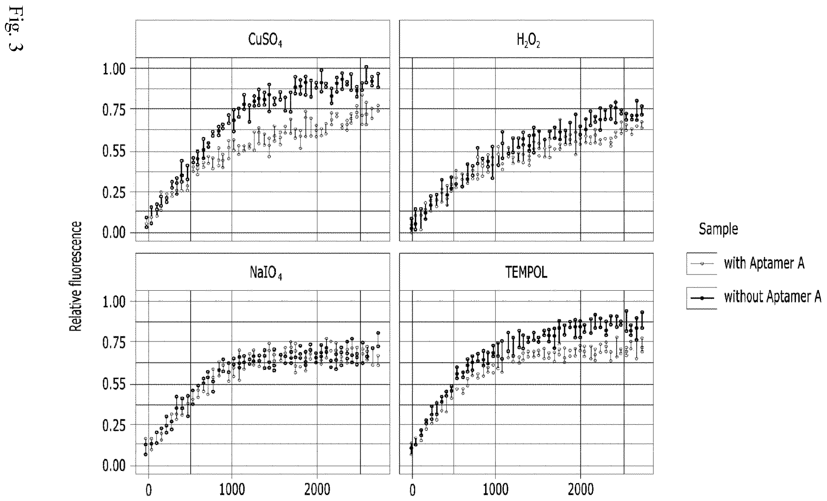

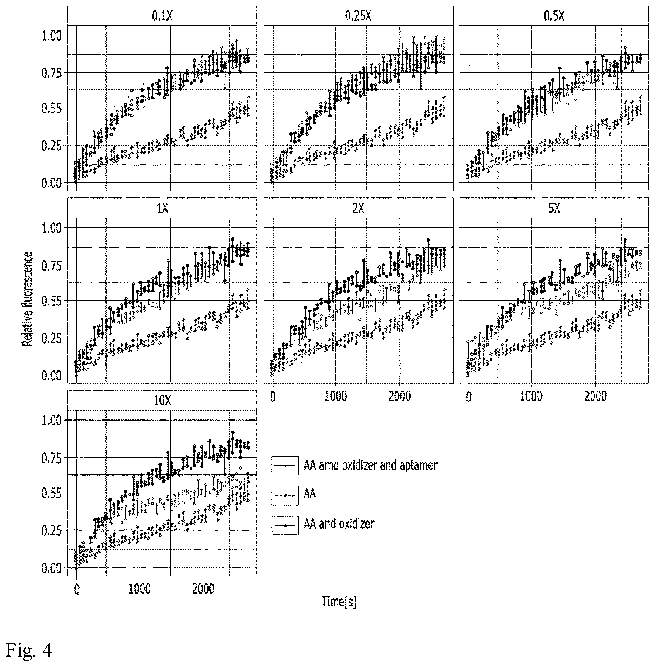

The ability to protect AA for the four (4) oxidants of aptamer A (CuSO4 (10.3 µM), NaIO4 (103.3 µM), H2O2 (10.3 µM) and TEMPOL (103.3 µM)) was evaluated. Titration of each oxidant was performed on AA (10.3 µM) (data not shown) to determine the lowest concentration to make an appropriate test window between the positive and negative controls. Aptamer 22 failed to protect against oxidation in the presence of NaIO4 (Figure 3, top right), but showed a reduction in the relative oxidation of AA of 19 ± 6% and 51 ± 7%, respectively, for H2O2 and TEMPOL (Figure 4, bottom panels). In addition, the re-analysis (with increased device settings) for CuSO4 showed 54 ± 5% oxidation reduction (Fig. 3, top left) (n = 3), which is 19% or more than the initial screening.

### Aptamer A Titration to AA

Aptamer A was incubated with ascorbic acid at relative concentrations of 10x, 5x, 2x, 1x, 0.5x, 0.25x and 0.lx. The titration data were drawn for the positive and negative controls (Fig. 4). Titration was applied in a dose-dependent manner. The oxidation protection effect was not observed at the lowest two concentrations (0. lx and 0.25x), and the oxidation protection effect was increased as the proportion of aptamer to AA is increased. The degree of oxidative protection did not show a steady state in the tested range, and thus the maximum protection effect of AA by the aptamer A occurred at a concentration of 10x or more of AA.

### Aptamer affinity measurement

The binding affinity of aptamer A to AA and DHA was measured by MST, and the detected KD values were 987.9 nM and 189.7 nM for AA and DHA, respectively (n = 3).

### Oxidation protection of AA in vitaminwater^{®}

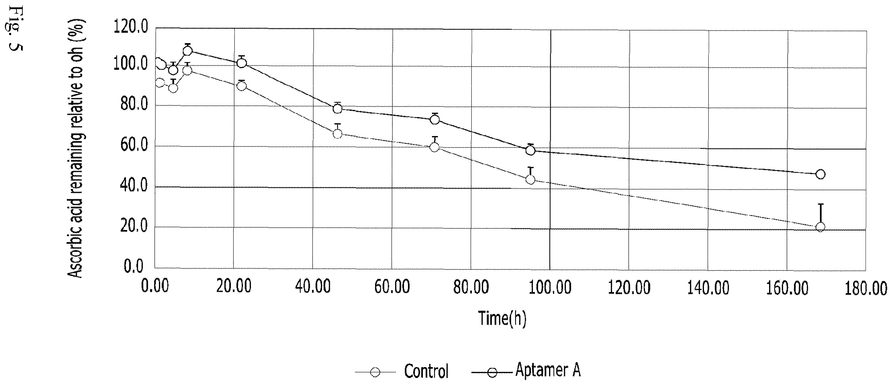

Data integration and quantification were performed with MultiQuant software (ABSciex) using area under the curve (AUC). AA-based quantification was measured for the time lapse of 168 h (FIG. 5). At the end of the time lapse, 21.9 ± 6.6% and 47.5 ± 0.5% of the initial AA remained (n = 3) in the control and the aptamer A - treated samples, respectively. The half-lives of A-A were 84 ± 10 h and 141 ± 14 h, respectively, in the control and aptamer A - treated samples. This means that the addition of aptamer A increases the half-life of AA by 168% in vitaminwater^{®}.

Also, since the vitamin beverage is pH 2.5, this means that the aptamin is also active at this pH.

### Example 6: Construction of a group of aptamers binding to reduced vitamin C

The systematic evolution of ligands by exponential enrichment (SELEX) was carried out under the following conditions, finding the aptamer which selectively binds to the reduced ascorbic acid from DNA aptamer library including 10¹³ of aptamers. To proceed with SELEX while the reduced state of ascorbic acid was maintained, glutathione was added while maintaining a pH of about 5.5.

Under these conditions, more than 99% of the vitamin C was maintained in the reduced L-ascorbic acid state, rather than the oxidized dehydro ascorbic acid (DHA). Under the above reaction conditions, SELEX was carried out, and the entire selected aptamers were subjected to Next Generation Sequencing. As an analysis result, aptamers composed of 3000 or more of secondary structure group were obtained.

### Example 7: Quantitative analysis of anti-oxidation of vitamin C by aptamer

Twenty individual aptamers were selected according to the type of the secondary structure, and the experiment regarding prevention of oxidation of vitamin C was carried out. After the aptamer dissolved in the annealing buffer was heated to 95°C, the secondary structure of the aptamer was formed as the temperature was gradually lowered to room temperature. Then, the mixture was mixed and reacted with the reduced L-ascorbic acid for 30 minutes so as to allow the mixture to bind to the L-ascorbic acid. Then, hydrogen peroxide solution was added to provide the oxidation condition. Oxidation of L-ascorbic acid was measured by adding OPDA (o-phenylenediamine) as a fluorescent dye. The level of DHA production can be quantitatively analyzed by measuring the amount of fluorescence from DHA-OPDA produced by the reaction of DHA, an oxide of L-ascorbic acid, with OPDA. Under the above conditions, the amount of fluorescence of DHA-OPDA was measured every 34 seconds for 25 minutes.

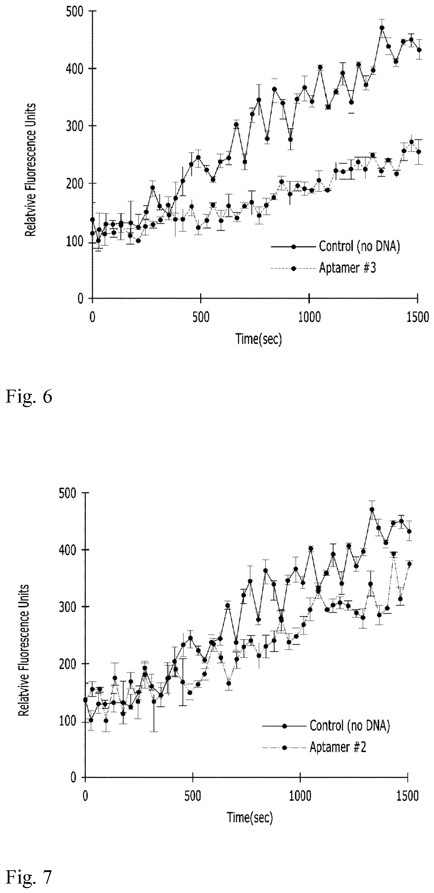

All three aptamers among them prevented oxidation of vitamin C by hydrogen peroxide water. The #12, #28, and #10 aptamers, respectively, prevented oxidation of about 40%, about 20%, and about 40%. Based on these experiments and other experiences, it can be concluded that the three aptamers react directly to vitamin C and thus prevent oxidation of vitamin C (*See* FIGS. 6 to 8).

### Example 8: Determination of steady-state solution dissociation constant (KD) of aptamer of the present disclosure for ascorbic acid (AA) and dehydroascorbic acid (DHA)

The dissociation constants were determined using microscale thermophoresis (MST).

The present Example includes MST data and KDs computed for the three aptamers in assay buffers for both targets. All reagents, including ascorbic acid and dehydroascorbic acid, were purchased from Sigma-Aldrich (St. Louis, MO), and deionized water was treated with Chelex-100 resin for 1 hour to remove accidental metals prior to buffer preparation. After Chelex treatment, the water was sprayed with nitrogen gas for 10 minutes to minimize oxygen, and then kept sealed. This water was used in all aqueous solutions. The final buffer included 50 mM sodium acetate, pH 5.5, 1 mM MgCl₂, and 0.05% Tween-20. Both AA and DHA were analyzed in 1:1 passages dilution from e5 µM to 153 pM (final) (for aptamers # 10 & # 12) and from 50 µM to 1.53 nM (final) (for aptamer # 8), respectively in buffer. The final concentration of each Cy5-conjugated aptamer is 20 nM.

Each technical second dilution was measured twice on a Monolith NT.115 MST device from NanoTemper Technologies GmbH (Munich, Germany).

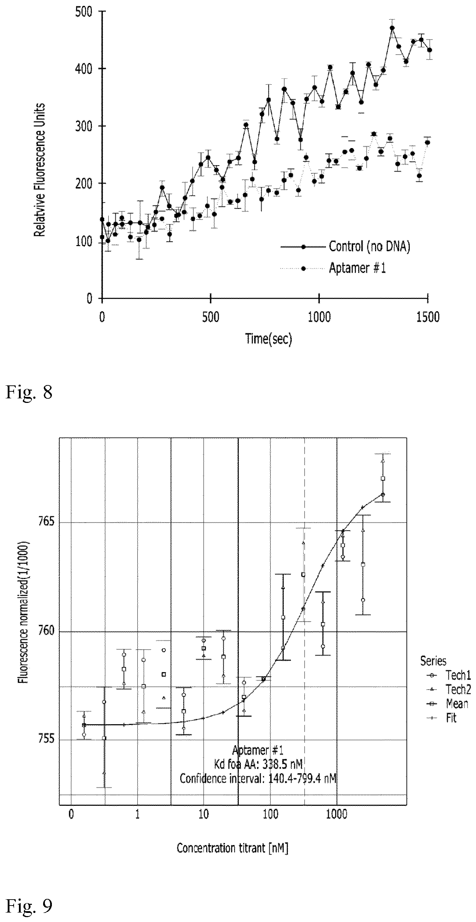

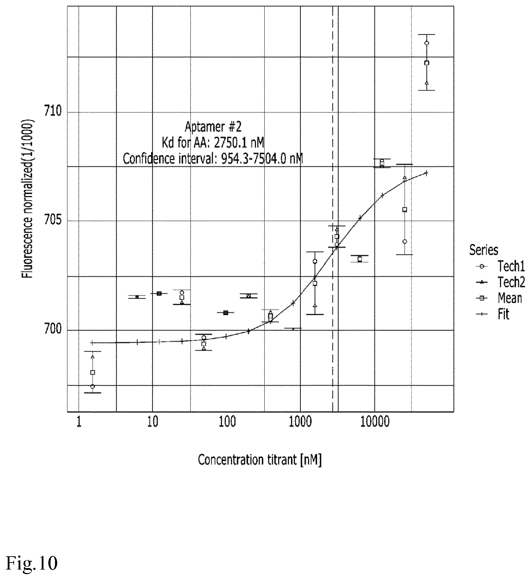

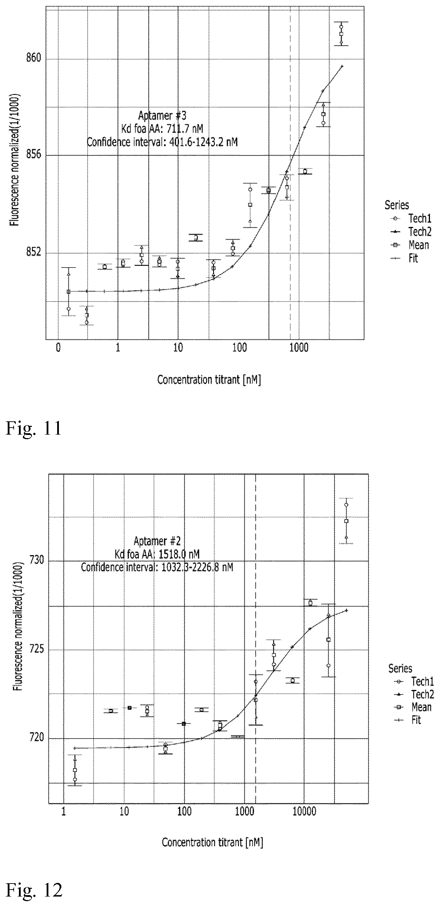

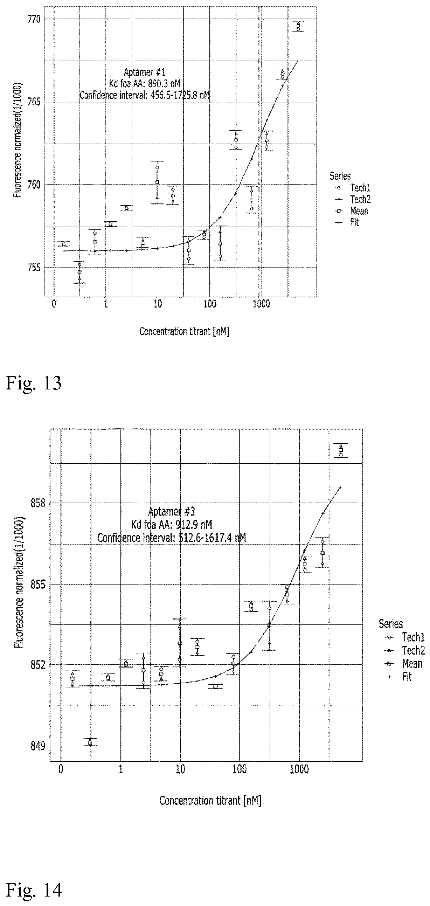

The results are shown in FIGS. 9 to 14. Each data point is shown with a mean and fitted curve (black line) in the drawings. The vertical dashed line in each graph represents KD.

The following conclusions were deduced from the above results.
1) aptamers #2 and #1 have better selectivity for AA vs. DHA, while aptamer #8 has slightly better selectivity for DHA than AA.
2) aptamer #3 has the best selectivity for AA vs DHA among the three aptamers.
3) aptamer # 1 was the best for the protection of AA from oxidation, but had the minimum selectivity for AA vs DHA.

### Example 9: Trapping of aptamer on hydrogel

The method of trapping the aptamer of the present disclosure on the hydrogel is a way to trap hydrothermal particles in the hydrogel by first attaching biotin to the amine group attached to the aptamer, then functionalizing them with the particle having streptavidin binding thereto, and then mixing them at a rate of 20% on calcination of the hydrogel. This method does not cause chemical bonding between the aptamer and the hydrogel, and it is relatively simple to implement.

Another method is to chemically attach the amine or carboxy group attached to the aptamer to the hydroxyl group of the hydrogel, which chemically binds the aptamer to the hydrogel.

In addition, various types of bonds can be brought by modifying the chemical composition of the hydrogel, or by changing the functional group to be bonded to the aptamer.

The specific trapping method includes the steps of: a) binding the amine group to the aptamer; b) silanizing the hydroxyl group of the hydrogel monomer with 3-glycidoxypropyltrimethoxysilane (3-GPTMS) having an epoxy group, and then binding amine group binding to the aptamer to the epoxy group; and c) polymerizing the hydrogel monomer, so that the aptamer-trapped hydrogel can be prepared.

Meanwhile, a collagen hydrogel, which is currently widely used due to moisturizing effects, can be made into an aptamer-collagen hydrogel in the same manner as described above, and it can add a function of more slowly releasing ingredients after sensing (detecting). For example, a smart sensing (detecting) function can be added so that teprenone or caprylic acid, which is used to prevent skin aging, is gradually supplied to the skin with an aptamer-collagen hydrogel or is released according to the amount of cytokine related to skin aging, which is released from the cell.

### Example 10: Production of Vitamin C-containing beverage based on aptamer of the present disclosure

To 1000 g of sterilized purified water was added 1 g of the aptamer vitamin C complex of the present disclosure, 500 g of oligosaccharide, 2 g of glycine, 2 g of taurine and 0.2 g of sodium citrate, and were mixed, and then the purified water was added again to make 2000 g of total weight.

Then, the solution to which the above components were added was stirred at 80 rpm for 1 hour, and filled in a vessel in a vacuum state to prepare a vitamin beverage containing vitamin C combined with the aptamer.

As a comparative example, the beverage was prepared under the condition that all the procedures were the same as those of Example 10, except that 1 g of vitamin C (UK DSM) was used instead of the aptamer vitamin C complex of the present disclosure.

### Experimental Example 1: Sensory evaluation

Sensory tests such as taste, aroma, and overall acceptability of the vitamin beverages of the present disclosure prepared in the above Example 10 and the beverages of the comparative example were measured, and the results are shown in Table 1 below.

In the above, the sensory test was conducted by 20 persons (10 males and 10 females, respectively) who were engaged in the food-related field for 3 years or more and it is that which is measured by the 5-point scale method. The numerical values for each item were obtained by dividing the total score of the sensory test agents by the number of sensory test persons and then rounded off to the second decimal place.

**[Table 1]**

| Item | Taste | Flavor | Overall palatability |
|---|---|---|---|
| Example 4 | 4.1 | 4.1 | 4.1 |
| Comparative Ex. | 4.0 | 4.0 | 4.0 |

Table 1 shows the sensory evaluation results of the beverage of the Example and the beverage of Comparative Example.

The sensory test means that the higher the value of the sensory test, the better the sensuality.

As can be seen from the above Table 1, the conventional vitamin beverages and the vitamin beverages of the present disclosure showed little difference in the sensory test, but the beverage of the present disclosure showed slightly higher test results.

### Experiment 2: Fatigue-improving effect of the beverage of the present disclosure

A questionnaire about the effect of fatigue improvement was obtained by taking each 150 ml of the vitamin beverages of the present disclosure prepared in the above Example 4 and the beverages of the comparative example only for 2 weeks on 50 persons (20 males and 30 females) with chronic fatigue symptoms, and the results are shown in Table 2 below.

**[Table 2]**

| Item | A | B | C | Total (persons) |
|---|---|---|---|---|
| Example 4 | 45 | 3 | 2 | 50 |
| Comparative Ex. | 35 | 10 | 5 | 50 |

Table 2 shows the fatigue-improvement effect (unit: persons) of Example 4 and Comparative Example, and in the table,
A: Having Fatigue-improving effect, B: No Fatigue improving effect, C: Not sure.

As shown in Table 2 above, all the vitamin beverages prepared in Example 10 and the beverages of the comparative examples prepared by the conventional method had fatigue-improving effects, but the fatigue-improving effect of the vitamin beverages prepared in Example 4 of the present disclosure was better than that of the beverage of the comparative example. This suggests that the beverage of the present disclosure is more effective in improving fatigue through maintenance of the antioxidative activity of vitamin C.

## Claims

1. Aptamer preventing oxidation of vitamin C, wherein the aptamer includes a base sequence as set forth in SEQ ID NO:1 or SEQ ID NO:2.

2. A method of preventing oxidation of vitamin C by treating vitamin C with an aptamer according to claim 1.

3. A method for preparing an aptamer-trapped hydrogel, the method comprising the steps of:
a) binding an amine group to the aptamer of claim 1;
b) silanizing a hydroxyl group of a hydrogel monomer with 3-glycidoxypropyltrimethoxysilane (3-GPTMS) having an epoxy group, and then binding an amine group binding to the aptamer to the epoxy group; and
c) polymerizing the hydrogel monomer.

4. The method of claim 3, wherein the method includes the step of binding a biotin to the amine group binding to the aptamer of the step a), then reacting the same with a particle having streptavidin, and then mixing the particle having the aptamer with the hydrogel monomer on hydrogel polymerization reaction.

5. The method of claim 3, wherein the hydroxy group of the hydrogel is bound to the amine group or a carboxyl group attached to the aptamer by a chemical method.

6. The method of any of claims 3 to 5, wherein the aptamer is attached to a surface of the hydrogel, and a specific ingredient is attached to an end of the aptamer.

7. The method of claim 6, wherein the specific ingredient is an ingredient having effects of skin aging prevention, wrinkle removal, whitening, or moisture.

8. A cosmetic composition comprising the aptamer-trapped hydrogel prepared according to the method of any of claims 3-5.

9. A non-therapeutic method for controlling release of a skin active material according to an amount of a target material released from a skin, comprising the steps of:
applying the aptamer-trapped hydrogel prepared according to the method of any of claims 3-5 to the skin to allow the aptamer binding to an ingredient from the hydrogel to penetrate into the skin; and
releasing the ingredient after binding the target material with the aptamer to which the ingredient is bound.

10. The method of claim 9, wherein the target material is ATP, wherein the release of the skin active material is controlled according to the amount of the target material released from the skin.

11. A food composition which comprises the aptamer preventing oxidation of antioxidant according to claim 1 as an effective ingredient.

12. The food composition of claim 11, wherein the food is selected from the group consisting of beverages, confectionaries, candies, dairy products, gums, soy sauces, breads, and ice cream.

## Patentansprüche

1. Aptamer, das die Oxidation von Vitamin C verhindert, wobei das Aptamer eine Basensequenz wie in SEQ ID NO:1 oder SEQ ID NO:2 dargelegt enthält.

2. Verfahren zur Verhinderung der Oxidation von Vitamin C durch Behandlung von Vitamin C mit einem Aptamer nach Anspruch 1.

3. Verfahren zur Herstellung eines mit einem Aptamer eingeschlossenen Hydrogels, wobei das Verfahren die folgenden Schritte umfasst:
a) Binden einer Aminogruppe an das Aptamer nach Anspruch 1;
b) Silanisieren einer Hydroxylgruppe eines Hydrogelmonomers mit 3-Glycidoxypropyltrimethoxysilan (3-GPTMS) mit einer Epoxygruppe und anschließendes Binden einer an das Aptamer gebundenen Amingruppe an die Epoxygruppe; und
c) Polymerisieren des Hydrogelmonomers.

4. Verfahren nach Anspruch 3, wobei das Verfahren den Schritt des Bindens eines Biotins an die Amingruppe, die an das Aptamer des Schritts a) bindet, dann das Reagieren desselben mit einem Teilchen, das Streptavidin enthält, und dann das Mischen des Teilchens, das das Aptamer enthält, mit dem Hydrogelmonomer bei der Hydrogelpolymerisationsreaktion umfasst.

5. Verfahren nach Anspruch 3, wobei die Hydroxygruppe des Hydrogels an die Amingruppe oder Carboxylgruppe, die mit dem Aptamer verbunden ist, durch ein chemisches Verfahren gebunden wird.

6. Verfahren nach einem der Ansprüche 3 bis 5, wobei das Aptamer an eine Oberfläche des Hydrogels gebunden ist und ein spezifischer Inhaltsstoff an ein Ende des Aptamers gebunden ist.

7. Verfahren nach Anspruch 6, wobei der spezifische Inhaltsstoff ein Inhaltsstoff ist, der Wirkungen der Hautalterungsprävention, der Faltenentfernung, der Aufhellung oder der Feuchtigkeitsspende hat.

8. Kosmetische Zusammensetzung, umfassend das Aptamerumschlossene Hydrogel, hergestellt nach dem Verfahren eines der Ansprüche 3 bis 5.

9. Ein nicht-therapeutisches Verfahren zur Kontrolle der Freisetzung eines hautaktiven Materials in Abhängigkeit von einer Menge eines Zielmaterials, das von der Haut freigesetzt wird, umfassend die folgenden Schritte:
Auftragen des gemäß dem Verfahren nach einem der Ansprüche 3 bis 5 hergestellten, mit Aptamer umschlossenen Hydrogels auf die Haut, um das Eindringen des Aptamers, das an einen Bestandteil des Hydrogels bindet, in die Haut zu ermöglichen; und
Freisetzen des Inhaltsstoffs nach Bindung des Zielmaterials mit dem Aptamer, an das der Inhaltsstoff gebunden ist.

10. Verfahren nach Anspruch 9, wobei das Zielmaterial ATP ist, wobei die Freisetzung des hautaktiven Materials entsprechend der Menge des von der Haut freigesetzten Zielmaterials kontrolliert wird.

11. Lebensmittelzusammensetzung, die das Aptamer, das die Oxidation des Antioxidans nach Anspruch 1 verhindert, als wirksamen Bestandteil enthält.

12. Lebensmittelzusammensetzung nach Anspruch 11, wobei das Lebensmittel aus der Gruppe bestehend aus Getränken, Süßwaren, Bonbons, Milchprodukten, Kaugummis, Sojasaucen, Broten und Eiscreme ausgewählt ist.

## Revendications

1. Aptamère empêchant l'oxydation de la vitamine C, dans lequel l'aptamère comporte une séquence de bases telle que présentée dans SEQ ID NO:1 ou SEQ ID NO:2.

2. Procédé d'empêchement d'oxydation de la vitamine C en traitant la vitamine C avec un aptamère selon la revendication 1.

3. Procédé de préparation d'un hydrogel piégé par un aptamère, le procédé comprenant les étapes :
a) de liaison d'un groupe amine à l'aptamère selon la revendication 1 ;
b) de silanisation d'un groupe hydroxyle d'un monomère d'hydrogel avec du 3-glycidoxypropyltriméthoxysilane (3-GPTMS) ayant un groupe époxy, puis de liaison d'un groupe amine se liant à l'aptamère au groupe époxy ; et
c) de polymérisation du monomère d'hydrogel.

4. Procédé selon la revendication 3, dans lequel le procédé comporte l'étape de liaison d'une biotine au groupe amine se liant à l'aptamère de l'étape a), puis de réaction de celle-ci avec une particule contenant de la streptavidine, puis de mélange de la particule contenant l'aptamère avec le monomère d'hydrogel lors de la réaction de polymérisation d'hydrogel.

5. Procédé selon la revendication 3, dans lequel le groupe hydroxyle de l'hydrogel est lié au groupe amine ou à un groupe carboxyle attaché à l'aptamère par un procédé chimique.

6. Procédé selon l'une quelconque des revendications 3 à 5, dans lequel l'aptamère est attaché à une surface de l'hydrogel, et un ingrédient spécifique est attaché à une extrémité de l'aptamère.

7. Procédé selon la revendication 6, dans lequel l'ingrédient spécifique est un ingrédient ayant des effets de prévention du vieillissement cutané, d'élimination des rides, de blanchiment ou d'hydratation.

8. Composition cosmétique comprenant l'hydrogel piégé par un aptamère préparé selon le procédé selon l'une quelconque des revendications 3 à 5.

9. Procédé non thérapeutique pour commander la libération d'un matériau actif cutané en fonction d'une quantité d'un matériau cible libéré par une peau, comprenant les étapes :
d'application de l'hydrogel piégé par un aptamère préparé selon le procédé selon l'une quelconque des revendications 3 à 5 sur la peau pour permettre à l'aptamère se liant à un ingrédient de l'hydrogel de pénétrer dans la peau ; et
de libération de l'ingrédient après la liaison du matériau cible avec l'aptamère auquel l'ingrédient est lié.

10. Procédé selon la revendication 9, dans lequel le matériau cible est l'ATP, dans lequel la libération du matériau actif cutané est commandée en fonction de la quantité du matériau cible libéré de la peau.

11. Composition alimentaire qui comprend l'aptamère empêchant l'oxydation de l'antioxydant selon la revendication 1 comme ingrédient efficace.

12. Composition alimentaire selon la revendication 11, dans laquelle l'aliment est choisi dans le groupe constitué de boissons, confiseries, bonbons, produits laitiers, gommes, sauces soja, pains et crèmes glacées.
